# EUROPEAN PATENT APPLICATION

(11) **EP 1 469 106 A1**
(43) Date of publication of application: **20.10.2004**
(21) Application number: 04007782.8
(22) Date of filing: 31.03.2004
(51) Int. Cl.: D01F 8/04, D01F 8/06, D01F 8/14, D04H 3/16, A61F 13/56

(54) **High-loft spunbond non-woven webs and method of forming same**

(30) Priority: 14.04.2003 US 413057
(71) Applicant: NORDSON CORPORATION, Westlake, Ohio 44145-1119 (US)
(72) Inventor: Allen, Martin A., Dawsonville Georgia 30534 (US); Crane, Patrick L., Dawsonville Georgia 30534 (US); Butt, Jon R. Sr., Atlanta Georgia 30306-3234 (US)
(74) Representative: Eisenführ, Speiser & Partner

(57) **Abstract**

A non-woven web formed from substantially continuous and uninterrupted multi-component filaments in which the filaments are spun at a spinning speed adequate for molecularly orienting less than all of the various constituent polymers. After collection, the non-woven web is either heated or receives a tensile force that causes each filament region formed from the polymer lacking molecular orientation to shrink in length considerably more than other filament regions formed from molecularly-oriented polymers. The differential length reduction enhances the loft of the non-woven web.

## Description

### Field of the Invention

The present invention relates generally to melt-spinning methods and products, and more particularly to methods of forming high-loft non-woven webs from multi-component filaments and high-loft non-woven webs formed by such methods.

### Background of the Invention

Melt spinning technologies are routinely employed to fabricate non-woven webs and multilayer laminates or composites. Melt spinning technologies, including spunbonding processes and meltblowing processes, form non-woven webs and composites from one or more layers of intertwined filaments or fibers, which are composed of one or more thermoplastic polymers. Certain nonwoven webs and composites are formed by a melt-spinning process known as spunbonding, which involves melt spinning of a thermoplastic polymer. The spunbonding process generally involves extruding fine diameter, semi-solid fibers or filaments of one or more thermoplastic polymers from multiple rows of orifices in a spinneret of a melt spinning apparatus. A flow of relatively cool process air is directed at the stream of extruded filaments to quench the molten thermoplastic polymer. A high-velocity flow of process air is then used to attenuate or draw the filaments to a specified diameter and to orient them on a molecular scale. The attenuated filaments are propelled in a filament/air mixture toward a forming zone to form a non-woven web or a layer of a laminate on a moving collector.

For certain applications, the spunbond filaments are formed from two or more thermoplastic polymers arranged in distinct regions across the cross-section of a multi-component filament. Multi-component spunbond filaments are extruded using flow passageways arranged to create flow paths for directing the individual polymers separately through the spinneret. Most frequently, multi-component filaments are extruded using two different polymers and, therefore, are more specifically referred to as bicomponent filaments.

Spunbond non-woven webs and laminates having a spunbond layer are incorporated into multiple different consumer and industrial products, such as various components of single-use or short-life hygienic articles, disposable protective apparel, fluid filtration media, and durables including bedding and carpeting. In particular, hygienic article components may be conveniently and efficiently manufactured using melt-spinning techniques. Hygienic articles generally include a liquid-pervious top sheet, a liquid-impervious back sheet, and a liquid storage layer positioned between the top sheet and the back sheet. An intervening fluid distribution layer may be positioned between the liquid storage layer and the top sheet. Each of these article components may comprise a spunbond non-woven web or a spunbond layer in a laminate.

Most hygienic articles are open-type and foldable for fastening to a wearer to form a three-dimensional pant configuration with a waist opening and a pair of leg openings. The hygienic article is maintained in the pant configuration by one or more closure elements, such as a tape strips or hook-and-loop fasteners. The hygienic article includes film landing zones effective for establishing a temporary adhesive bond with the tape strips. Typically, the landing zone is dimensioned so that the hygienic article can be secured to wearers of different body sizes.

Hook-and-loop fasteners represent an advancement in fastening a disposable hygienic article to the wearer. The loop-type and hook-type fasteners are strategically positioned at different spatial locations on the hygienic article so that the article can be fastened in the three-dimensional pant configuration to the wearer. Typically, the loop-type fastener is dimensioned to operate as a landing zone so that the hygienic article can be secured to wearers of different body sizes. However, hook-and-loop fasteners are expensive to manufacture and contribute significantly to the cost of the hygienic article.

Alternatively, hygienic articles may be manufactured that are pre-shaped as three-dimensional articles. Such articles lack closure elements. However, in this situation, the hygienic article must have a stretchable pull-up design so that either one size fits all or a few sizes fit all.

Landing zones consisting of loop-type fasteners have been formed conventionally from spunbond filaments. Such filaments are treated in an attempt to provide a structure with sufficient loft to operate as a landing zone of loop material. For example, the surface may be brushed with a mechanical brush. However, treatment by brushing adds an extra step and cost to the manufacturing process. Another attempt to add loft is to sheer conventional filaments with a sharp knife. However, a landing zone formed in this manner does not fasten with an effective bond to the hook member and also adds an extra manufacturing step and cost. Another conventional method is to use a high denier polymer as a landing zone. However, high denier polymers are expensive to produce and may produce wearer discomfort as such non-woven materials are stiff and rough.

Loop-type fasteners used for conventional landing zones are typically larger than the complementary hook-type fastener so that some relative positioning error is permitted when securing the two components together. However, the allowable positioning error may be insufficient to guarantee that the hook-type fastener will contact the loop-type fastener in a single fastening attempt. In addition, the hook-type fastener may have only a partial contact with the loop-type fastener after fastening that is insufficient to keep the hygienic article secure to the wearer. After being soiled, hygienic articles are removed from the wearer and discarded. When removed from the wearer, solid waste held by the hygienic article is no longer constrained and may be released into the surrounding environment, which represents a significant problem with conventional hygienic articles.

For these reasons, it is desirable to provide a melt-spinning method for forming a high-loft non-woven web and a high-loft non-woven web formed by this melt-spinning method.

### Summary

The present invention addresses these and other problems associated with the prior art by providing a method of making a non-woven web having a high loft and products formed by the method. The method includes forming a plurality of substantially continuous and uninterrupted multi-component filaments from at least a first polymer and a second polymer, in which each of the filaments has at least distinct first and second regions comprising the first polymer and the second polymer, respectively. The filaments may be formed by any conventional spinneret or by melting each polymer component and combining. The filaments are attenuated at a spinning speed effective for causing significant molecular orientation of only the first polymer. The second polymer region is caused to shrink such that a reduction in length of the second polymer region is greater than a reduction in length of the first polymer region.

In one application of the invention, the non-woven web of multi-component filaments may operate as a loop-type material that cooperates with a complementary hook-type material of any common hook-and-loop closure element. The hook-and-loop fastener incorporating the loop-type material of the invention may be used, for example, to secure a hygienic article to a wearer. In another specific application relating to hygienic articles, the non-woven web of multi-component fibers is used to construct an outermost layer of a back sheet so that a hook-type fastener can be secured with any specific surface area of the back sheet. The hook-type fastener is no longer constrained to engaging a relatively small rectangular strip of loop-type material.

The invention is not limited to use for forming components of hygienic articles. In applications other than for hygienic articles, the crimped filaments of the invention may be used as high loft stuffing material in comforters and mattress ticking, and as insulation in jackets or insulative fill. Moreover, the crimped filaments of the invention may be used in air filtration products to provide a tortuous air path for filtering particles out of an air stream.

According to the principles of the invention, the non-woven web of multi-component filaments of the invention does not require any secondary treatments, such as brushing or shearing, to provide sufficient loft to have adequate functionality for its intended applications.

These and other objects and advantages of the present invention shall become more apparent from the accompanying drawings and description thereof.

### Brief Description of the Figures

The accompanying drawings, which are incorporated in and constitute a part of this specification, illustrate embodiments of the invention and, together with a general description of the invention given above, and the detailed description given below, serve to explain the principles of the invention.
Fig. 1 is a perspective view of a hygienic article incorporating a landing zone constructed from a non-woven web of multi-component filaments in accordance with the principles of the invention;
Fig. 2 is a perspective view of a hygienic article incorporating a back sheet constructed from a non-woven web of multi-component filaments in accordance with the principles of the invention; and
Fig. 3 is a diagrammatic view of a spunbonding system capable of producing the non-woven web of the invention.

### Detailed Description of the Invention

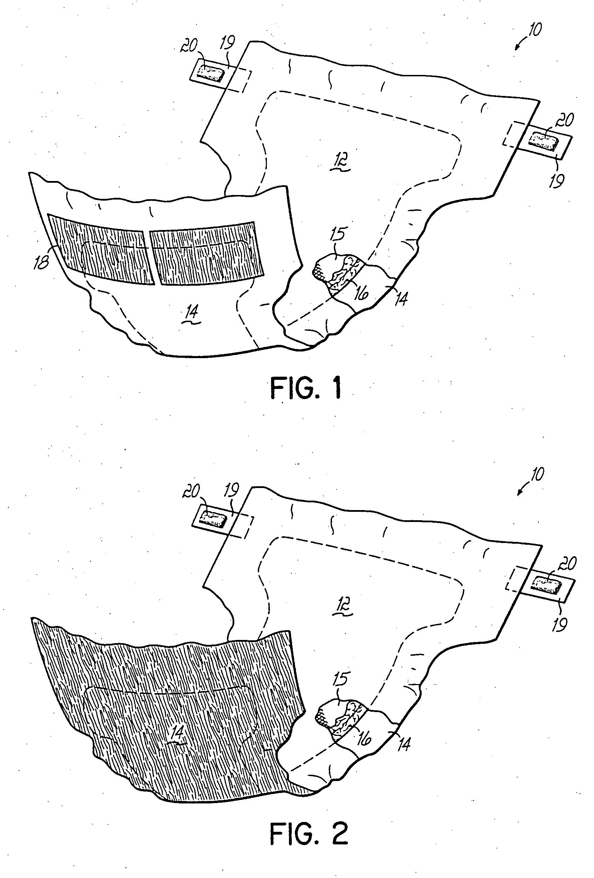

With reference to Fig. 1, a disposable hygienic article 10 generally includes a top sheet 12, a back sheet 14, and a liquid storage layer 16 positioned between the top sheet 12 and the back sheet 14. The top sheet 12 transfers aqueous body fluids, such as urine, to the liquid storage layer 16. Hygienic article 10 includes a fluid acquisition and transfer layer 15 between the liquid storage layer 16 and top sheet 12 that allows full utilization of the fluid capacity of the underlying liquid storage layer 16. The liquid storage layer 16 includes an absorbent material capable of absorbing large quantities of aqueous body fluids and retaining the absorbed fluids under moderate applied pressures. The top sheet 12 is fluid pervious so that aqueous body fluids may readily penetrate through its thickness to the liquid storage layer 16. The back sheet 14 prevents aqueous body fluids absorbed and contained in the liquid storage layer 16 from wetting articles present in the surrounding environment, such as pants, pajamas and undergarments.

Hygienic article 10 includes a pair of closure elements each consisting collectively of a loop-type fastener 18 attached to the back sheet 14 and a hook-type fastener 20 attached to a corresponding attachment tab 19 extending away from the back sheet 14. The loop-type fastener 18 is formed of a loop-type material that includes a plurality of loop members extending outwardly from a backing structure. The hook-type fastener 20 is formed of a hook-type material having a plurality of hook members extending outwardly from a backing structure. The loop-type fastener 18 operates as a landing member or zone and the hook-type fastener 20 operates as an attachment member or zone that is releasably anchorable or attachable to the loop-type member 18. According to the principles of the invention, the loop-type fastener 18 is formed from a plurality of substantially continuous and uninterrupted multi-component filaments including at least two distinct polymer constituents or components in which the spinning speed chosen for attenuation is effective to crystallize one of the two polymers and is insufficient to provide full crystallization of the other polymer.

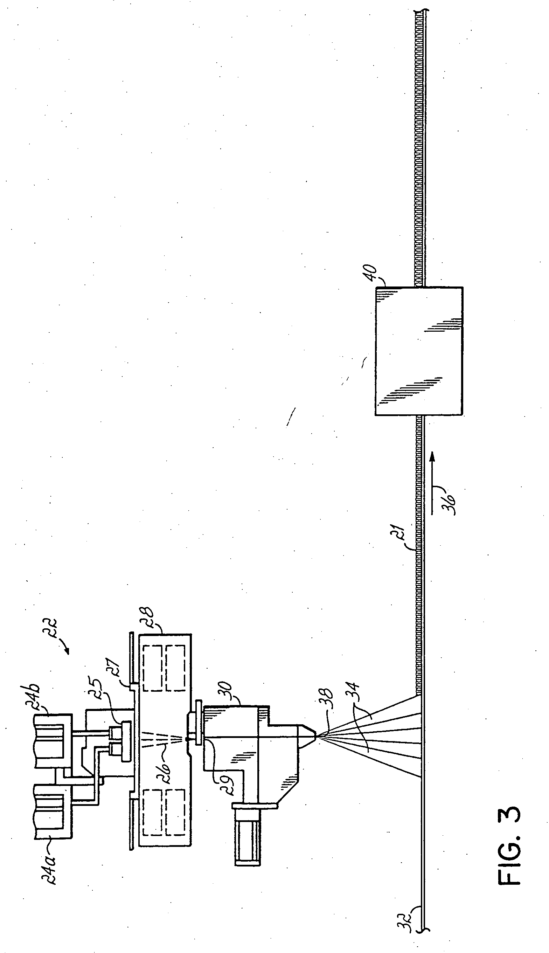

With reference to Fig. 3, a melt spinning apparatus 22 includes a spinneret 25 capable of producing substantially continuous and uninterrupted filaments 26 having at least two distinct polymer regions. Spinnerets 25 capable of extruding filaments 26 are described, for example, in United States Patent No. 6,478,563, co-pending U.S. Application Serial No. 09/702,387 entitled "Apparatus for Meltblowing Multi-Component Liquid Filaments" and filed October 31, 2000, co-pending U.S. Application Serial No. 09/802,646 entitled "Apparatus and Method for Extruding Single-Component Liquid Strands Into Multi-Component Filaments" and filed March 9, 2001, and co-pending U.S. Application Serial No. 09/802,651 entitled "Apparatus for Extruding Multi-Component Liquid Filaments" and filed March 9, 2001. The disclosure of each of these documents is hereby incorporated by reference herein in its entirety.

Multi-component filaments suitable for forming the loop-type fastener 18 (Fig. 1) include, but are not limited to, sheath/core bicomponent arrangements in which one polymer forms a sheath about a core formed from the other polymer, side-by-side bicomponent configurations in which the two polymers are arranged side-by-side, and multi-lobal bicomponent configurations. The polymers constituting the filaments are arranged as at least two distinct polymer regions each comprising one of the polymers. The substantially continuous and uninterrupted multi-component filaments are prepared by coextruding, or by melting and combining, the constituent polymers as filaments having a round, oval, trilobal, triangular, dog-boned, flat or hollow shape. The polymers used to fabricate the multi-component filaments may be any commercially available spunbond grade of a wide range of thermoplastic polymeric materials including, without limitation, polyolefins, polyamides, polyesters, polyamides, polyvinyl acetate, polyvinyl chloride, polyvinyl alcohol, cellulose acetate, and the like. In one specific embodiment of the invention, one of the polymers is polyester and the other of the polymers is either polypropylene or polyethylene. The polyester typically has an intrinsic viscosity between 0.1 and 0.8, which is an indication of molecular weight.

The spinneret 25 receives streams of molten polymer from at least two melters 24a, 24b and combines the polymers to form a curtain of the thermoplastic filaments 26 that generally spans the width of a collector 32, such as a table or belt. The airborne curtain of filaments 26 passes through a monomer exhaust system 27 that evacuates any residual monomer gas from the extrusion process. The airborne curtain of filaments 26 next traverses a quenching system 28 that directs a flow of cool process air onto the curtain of filaments 26 for quenching the filaments 26 and initiating the solidification process.

With continued reference to Fig. 3, the airborne curtain of filaments 26 from quenching system 28 is directed by suction into an inlet 29 of a filament drawing device 30. The filament drawing device 30 envelops the filaments 26 with a tangential high velocity flow of process air that applies a biasing or tensile force in a direction substantially parallel to the length of the filaments 26. Because the filaments 26 are extensible, the high velocity flow of process air in the filament drawing device 30 attenuates and molecularly orients the filaments 26 to form attenuated filaments 34. The attenuated filaments 34 are entrained in the high velocity process air when discharged from an outlet 38 of the filament drawing device 30 toward the collector 32.

The spinning speed in the filament drawing device 30 is selected such that the attenuated filaments 34 are not crimped during attenuation. Instead, the attenuated filaments 34 possess latent crimp that is activated by post-collection treatment. Different polymers have different spinning speeds for which molecular orientation is achieved. For example, a threshold spinning speed of about 3500 meters per minute is required to impart significant molecular orientation to polypropylene, and a threshold spinning speed of about 5500 meters per minute is required to initiate molecular orientation of polyester. The degree of molecular alignment depends on the spinning speed, and the alignment of the polymer molecular chains is related to the degree of polymer crystallization, which directly relates to the filament stability.

Filaments 34 formed from improperly drawn polymers are prone to length shrinkage. Spinning speeds less than the threshold value to initiate molecular orientation provides under-developed molecular orientation and crystallization, which leads to significant shrinkage if the filament is subsequently heated or tension is applied. For example, spinning a bicomponent polymer filament at a spinning speed selected to molecularly orient only one of the two polymers creates a non-woven web of constituent filaments having latent crimp. It is appreciated by a person of ordinary skill in the art that each of the polymers may be oriented molecularly but that, in accordance with the principles of the invention, only one of the polymers constituting the attenuated filaments 34 has a substantial degree of molecular orientation.

With continued reference to Fig. 4, the attenuated filaments 34 are deposited or laid down on the collector 32 in a random manner to form the non-woven web 21. The non-woven web 21 is conveyed on collector 32 in a machine direction 36 to a post-collection treatment device 40. Processing the non-woven web 21 in the post-collection treatment device 40 activates the latent crimp of filaments 34. The web 21 is cut into rectangular portions, which are attached to a backing strip adhesively bonded to the attachment tabs 19 (Fig. 1) to form the loop-type fastener 18 of hygienic article 10.

Several different activation processes may be used for activating the latent crimp to produce the high-loft spunbond non-woven web 21. Regardless of the specific process relied upon for activating the latent crimp, the multi-component filaments 34 in the non-woven web 21 are bulked up by the activated crimp so as to enhance the loft.

In one activation process, treatment device 40 is a heating device, such as a dryer or an oven, that exposes web 21 to a heated environment of greater than about 100°F for a time sufficient to activate the latent crimp of attenuated filaments 34. The heat treatment operates for increasing the loft of the non-woven web 21 as compared with non-woven webs formed from single component non-woven webs of either individual polymer. The temperature and duration of the heating is effective to cause shrinkage of one of the two constituent polymers without causing degradation of either polymer. It is apparent that heating temperature and duration bear a reciprocal relationship in that lower heating temperatures require a lengthier heat treatment. The non-woven web 21 may be conveyed at a normal production line speed through the heating device.

In one specific embodiment of the invention, non-woven web 21 is formed from bicomponent polypropylene/polyester filaments 34 spun at a spinning speed of about 3500 to 4000 meters per minute, which is suitable to provide significant molecular orientation of the polypropylene but not polyester. In particular, the spinning speed is selected to be significantly less than the threshold spinning speed for polyester of about 5500 m/min. The resultant bicomponent filaments 34 possess latent crimp. Typically, the polypropylene and polyester polymers are arranged with a side-by-side relationship or in an asymmetric or eccentric sheath-core relationship in which polyester constitutes the core. When heated in treatment device 40, the polyester region of each filament 34 shrinks significantly and the polypropylene region maintains its original length or only experiences minor shrinkage. As a result, filaments 34 crimp due to the differential shrinkage. An effective temperature for activating crimp in bicomponent filaments of polyester/polypropylene is any temperature exceeding about 180°, which results in shrinkage of the polyester, and less than about 400°F to 450°F, which degrades polypropylene. Typically, such non-woven webs 21 are heated to a temperature in the range of about 250°F to about 350°F for activating the latent crimp.

Another treatment device 40 suitable for activating the latent crimp is an aqua jet that exposes the non-woven web 21 of multi-component filaments 34 to a plurality of water streams. The water streams impart stretching or tensioning forces into the non-woven web 21. The aqua jet includes a nozzle having multiple water-emitting orifices, typically having a density of about 30 to 50 orifices per inch, each emitting a high pressure stream or jet of water in the range of about 500 psi to about 1500 psi that penetrates through the non-woven web 21. The high speed of the water mechanically entangles and places significant stress and strain on the filaments 34, which activates the latent crimp. Aqua jets suitable for use in the invention are commercially available from Fleissner GmbH & Co. (Egelsbach, Germany).

With continued reference to Fig. 4, another treatment device 40 for activating the latent crimp is a tenter frame, which is a machine that dries non-woven web 21 while stretched in a cross-machine direction, generally perpendicular to machine direction 36 (Fig. 4), to a specified width under tension. The tenter frame consists of a heated chamber and a conveyor belt passing through the heated chamber. The conveyor belt has a clamping structure on each of its side edges extending parallel to the machine direction 36 in which the non-woven web 21 carried by the conveyor 32 is moving. The clamping structures diverge outwardly at an angle, for example 15°, relative to each other in the machine direction 36. The non-woven web 21 is transferred from the conveyor 32 to the conveyor belt and grasped by the clamping structure. The non-woven web 21 is stretched or widened by the movement through the tenter frame due to the outward divergence of the clamping structure. The non-woven web 21 is released from the tenter frame after stretching.

The tenter frame may, in the alternative, have a spaced apart pair of endless chains on horizontal tracks rather than a conveyor belt. The non-woven web 21 is held firmly at the edges by pins or clips on the two chains that diverge as they advance through the heated chamber, adjusting the non-woven web 21 to the desired width. The outward divergence stretches or tensions the constituent filaments 34 of the non-woven web 21 primarily in the cross-machine direction, which activates the latent crimp.

Yet another treatment device 40 capable of activating the latent crimp of filaments 34 consists of two sets of nip rollers that are spaced apart in the machine direction 36, in which the non-woven web 21 is transported through the nip rollers. The angular velocity of the two sets of nip rollers differs so that the trailing set of nip rollers rotates faster than the leading set of nip rollers. This difference in angular velocity applies a continuous stretch or tension in the machine direction 36 to the filaments 34. The applied tension activates the latent crimp of the filaments 34 for increasing the loft of the non-woven web 21.

With reference to Fig. 2 in which like reference numerals refer to like components in Fig. 1, the high-loft non-woven web 21 (Fig. 3) of the invention may also be used to construct the entire outermost layer of the back sheet 14 of hygienic article 10. In particular, the non-woven web 21 of the invention is used to form the outermost layer of a composite forming the back sheet 14. In this embodiment of the invention, the loop-type fastener 18 is eliminated as the entire back sheet 14 constitutes loop-type material to which the hook-type fastener 20 may be releasably engaged. This permits significant error in the positioning of the hook-type fastener 20 as the complementary loop-type material is no longer constrained to the dimensions of a rectangular backing strip (Fig. 1). In addition, forming the outermost layer of the entire back sheet 14 from the non-woven web 21 of the invention eases disposal of a soiled hygienic article 10 as the hygienic article may be simply rolled into a self-contained and closed ball.

To that end, the back sheet 14 may be formed by laminating the non-woven web of the invention to a layer of fluid-impervious material, such as polyethylene film. The fluid-impervious material acts as a liquid barrier and the non-woven web of the invention operates as a landing zone for the hook-type fastener 20. Alternatively, the back sheet 14 may also be formed as a composite consisting of the non-woven web of the invention and a layer of meltblown fibers that acts as a liquid barrier. The S/M composite is point-bonded with a heated calendar to provide strength. Alternatively, the back sheet 14 may be formed as an S/M/S composite including the non-woven web of the invention, a layer of meltblown fibers laid on the non-woven web of the invention, and another spunbond layer laid on the meltblown layer. Additional layers may be added to the S/M/S structure as understood by a person of ordinary skill in the art.

The non-woven web 21 (Fig. 3) of the invention may also be employed to manufacture fluid acquisition and transfer layer 15 (Figs. 1 and 2). The improved loft enhances the open porous structure of the fluid acquisition and transfer layer 15 so that aqueous body fluids originating from the wearer of the hygienic article 10 can penetrate rapidly and spread out for absorption by liquid storage layer 16. After absorption, the fluid acquisition and transfer layer 15 separates or isolates the top sheet 12 of the hygienic article 10 and, therefore, the article wearer's skin is not rewetted by the absorbed aqueous body fluids captured in the liquid storage layer 16. The high-loft of the fluid acquisition and transfer layer 15 operates to increase the separation by increasing the distance between the article wearer's skin and the liquid storage layer 16.

While the present invention has been illustrated by a description of various embodiments and while these embodiments have been described in considerable detail, it is not the intention of the applicants to restrict or in any way limit the scope of the appended claims to such detail. Additional advantages and modifications will readily appear to those skilled in the art. The invention in its broader aspects is therefore not limited to the specific details, representative apparatus and methods, and illustrative examples shown and described. Accordingly, departures may be made from such details without departing from the spirit or scope of applicants' general inventive concept. The scope of the invention itself should only be defined by the appended claims, wherein we claim:

## Claims

1. A method of making a non-woven web with a high loft, comprising:
melting a first polymer;
melting a second polymer;
combining the first and second polymers to form a plurality of substantially continuous and uninterrupted multi-component filaments, each of the filaments having distinct first and second regions comprising the first polymer and the second polymer respectively;
attenuating the filaments at a spinning speed effective for causing significant molecular orientation of only the first polymer;
collecting the plurality of substantially continuous and uninterrupted multi-component filaments to form a non-woven web; and
reducing a second length of the second region of each filament more than a first length of the first region.

2. The method of claim 1 wherein reducing the second length further comprises:
applying tension to the non-woven web after collection in an amount effective to cause the reduction of the second length.

3. The method of claim 2 further comprising:
applying tension using a device selected from the group consisting of a tenter frame, an aqua jet, and two sets of nip rollers spaced along a machine direction and operating at different angular velocities.

4. The method of claim 1 wherein reducing the second length further comprises:
heating the non-woven web after collection to a temperature and for a duration effective to cause the reduction in the second length.

5. The method of claim 4 wherein heating the non-woven web further comprises:
conveying the non-woven web through an oven.

6. The method of claim 1 wherein the first polymer is polypropylene and the second polymer is polyester, and the spinning speed is about 3500 meters per minute.

7. A high-loft spunbond non-woven web formed from a plurality of substantially continuous and uninterrupted multi-component filaments produced by the process comprising the steps of:
melting a first polymer;
melting a second polymer;
combining the first and second polymers to form a plurality of substantially continuous and uninterrupted multi-component filaments, each of the filaments having distinct first and second regions comprising the first polymer and the second polymer respectively;
attenuating the filaments at a spinning speed effective for causing significant molecular orientation of only the first polymer;
collecting the plurality of substantially continuous and uninterrupted multi-component filaments to form a non-woven web; and
reducing a second length of the second region of each filament more than a first length of the first region.

8. The non-woven web of claim 7 wherein the multi-component filaments have a sheath/core bicomponent arrangement, the first region being a core and the second region being a sheath surrounding the core.

9. The non-woven web of claim 8 wherein the core is formed from polyester and the sheath is formed from one of polyethylene and polypropylene.

10. The non-woven web of claim 7 wherein the multi-component filaments have a side-by-side bicomponent arrangement, the first polymer region forming a first side and the second polymer region forming a second side.

11. The non-woven web of claim 10 wherein the first polymer is selected from polyethylene and polypropylene and the second polymer is polyester.

12. The non-woven web of claim 7 wherein the non-woven web is used to construct a component of a hygienic article selected from the group consisting of a back sheet, a fluid acquisition and transfer layer, and a loop-type material capable of being releasably coupled with a hook-type material of hook-and-loop fastener.

13. A hygienic article comprising:
a liquid-pervious top sheet;
a liquid-impervious back sheet covering the liquid storage layer, said back sheet having an outwardly-facing surface formed from a loop-type material capable of forming a releasable attachment with a hook-type material;
a liquid storage layer positioned between said top sheet and said back sheet; and
at least one hook-type fastener attached to said back sheet, said hook-type fastener being formed from said hook-type material.
